# EUROPEAN PATENT APPLICATION

(11) **EP 4 321 095 A1**
(43) Date of publication of application: **14.02.2024**
(21) Application number: 22189956.0
(22) Date of filing: 11.08.2022
(51) Int. Cl.: A61B 5/16

(54) **DETECTION OF SUBTLE COGNITIVE IMPAIRMENT**

(71) Applicant: Koninklijke Philips N.V., 5656 AG Eindhoven (NL)
(72) Inventor: KLAMING, Laura, Eindhoven (NL); SPELT, Hanne Adriana Alijda, Eindhoven (NL); VAN EE, Raymond, 5656AG Eindhoven (NL)
(74) Representative: Philips Intellectual Property & Standards

(57) **Abstract**

A means for distinguishing subjects with subtle cognitive impairment in the context of a neuropsychological assessment which comprises a battery of cognitive tests. A first set of tests is administered which have a time-constraint or other cognitive demand parameter imposed. A second set of tests is administered for which the time constraint or other cognitive demand parameter is not imposed. Based on comparing or relating the scores between the two sets of tests, subtle cognitive impairment can be detected, based on a criterion that subjects with subtle cognitive impairment present with a greater disparity in these two sets of scores than do normal subjects.

## Description

### FIELD OF THE INVENTION

The present invention relates to the field of neuropsychological assessment.

### BACKGROUND OF THE INVENTION

Patients with subtle cognitive impairment, e.g. due to mild traumatic brain injury (mTBI) or multiple sclerosis (MS), often perform well on a standard neuropsychological assessment, such as the Philips ISC (IntelliSpace Cognition) test battery. The main reason for this is that standard outcome measures of cognitive tests are not sufficiently sensitive to detect subtle cognitive impairment. This is a problem because patients with subtle cognitive impairment may experience substantial difficulties in their everyday lives due to their subtle cognitive impairment, but will be considered healthy based on the outcome of their neuropsychological assessment.

### SUMMARY OF THE INVENTION

The invention is defined by the claims.

According to examples in accordance with an aspect of the invention, there is provided a system comprising:
an input/output; and
one or more processors adapted to execute a method, the method comprising:
   controlling, via control signals communicated though the input/output, a test apparatus to administer a battery of cognitive tests to a subject, wherein the battery of cognitive tests includes:
      a plurality of first tests, wherein each of the first tests is a test for which a time constraint is imposed related to a completion time for the test or for which another cognitive demand constraint is imposed, and
      a plurality of second tests, wherein each of the second tests is a test for which said time constraint is not imposed or said other cognitive demand constraint is not imposed;
   generating a first composite score from a combination of test scores obtained for the subject from the plurality of first tests;
   generating a second composite score from a combination of test scores obtained for the subject from the plurality of second tests;
   computing a characteristic value based on the first composite score and the second composite score;
   detecting presence of subtle cognitive impairment based on the characteristic value; and
   generating an output at the input/output indicative of the detection.

The general concept comprises the detecting of subtle cognitive impairment in a cognitive test battery by using composite scores for cognitive tests that have a certain cognitive demand constraint imposed and those that do not, for example those that are timed and those that are not timed.

With regards to the time constraint option, studies have shown that patients with subtle cognitive impairment have more difficulties performing cognitive tests that are time-dependent. This is most likely due to the fact that time-dependent tests impose additional cognitive demands making the test more difficult. Reference is made for example to the paper: Barrow, I.M., et al. (2003). Can within-category naming identify subtle cognitive deficits in the mild traumatic brain-injured patient? The Journal of Trauma, 54(5), 888-895. Reference is also made for example to the paper: Raskin, S.A., et al. (1998). Neuropsychological assessment of individuals with mild traumatic brain injury. The Clinical Neuropsychologist, 12(1), 21-30.

Thus, by administering both the first and second sets of tests (e.g. timed and untimed tests), and computing a characteristic value which depends upon the relative scores obtained for these two categories, more sensitive detection of subtle cognitive impairment is achieved. In particular, the expectation is that a difference between typical scores obtained for timed tests and typical scores obtained for untimed tests would be greater for those individuals with subtle cognitive impairment.

The same principle also holds for any other cognitive demand constraint which is added to one test and not another, aside from a time constraint. Other examples of cognitive demand constraints/parameters could include for instance: presence of environmental noise, imposition of a multitasking requirement, adjustment of visibility of test stimuli (e.g. brightness, clearness of stimuli, contrast of stimuli), imposition of tactile constraints (e.g. chair moving, pen difficult to hold). It is noted that in this disclosure the terms 'cognitive demand constraint' and 'cognitive demand parameter' are synonymous. Each relates to a parameter or property or aspect or constraint of the test which has the effect of imposing additional cognitive demand on the subject performing the test compared to if that parameter/property/aspect/constraint were not present.

The time constraint or other cognitive demand constraint presents a cognitive demand beyond the demands of the test itself.

In some embodiments, the scores for different tests may be normalized scores, such that scores from different tests are comparable with one another.

In some embodiments, the time-constraint comprises an instruction to the subject to complete the task as fast as possible, and/or comprises a time limit for completion of the task.

In some embodiments, the characteristic value comprises a ratio between the first composite score and the second composite score, or vice versa.

As noted above, a difference between the scores can be expected to be larger for people with subtle cognitive impairment. Thus a ratio is one simple way to represent the disparity between the composite scores.

For example, in some embodiments, subtle cognitive impairment is detected in the event that the ratio between the first composite score (e.g. timed tests) and the second composite score (e.g. untimed tests) falls outside of a pre-defined range, for example falls below a pre-defined threshold.

Since the first test scores will typically always be lower than the second test scores for both healthy and unhealthy individuals, this can be reflected by determining a ratio between the first composite score (e.g. time-constrained) and the second composite score (e.g. non time-constrained) and seeing if this falls below a pre-defined threshold. The greater the difference between the scores, the smaller this ratio becomes.

In some embodiments, the characteristic value comprises a difference between the second composite score and the first composite score.

In some embodiments, the one or more second tests include one or more of: Clock Drawing Test, Digit Span Test, Rey Auditory Verbal Learning Test, Rey Osterrieth Complex Figure Test.

In some embodiments, the one or more first tests include one or more of: Trail Making Test, Controlled Oral Word Association Test, Category Fluency Test, Star Cancellation Test, Symbol Digit Modalities.

In some embodiments, at least a subset of the battery of cognitive tests are configurable to either impose or not impose a time-constraint or other cognitive demand constraint when administering the test.

In some embodiments, the method comprises administering a same cognitive test with a time-constraint or other cognitive demand constraint imposed, to form one of the one or more first tests, and without the time constraint or other cognitive demand constraint imposed, to form one of the one or more second cognitive tests.

For example a same test is conducted with and without time pressure to increase or decrease cognitive demands, respectively. By imposing the same test with and without the time constraint or other cognitive demand constraint, this means that any difference between obtained scores for the two by an individual are more likely to be due to the time constraint or other cognitive demand constraint. In some embodiments, a correction might additionally be performed to the score for the version of the test which is performed second, to correct for learning effects.

In some embodiments, a procedure can be followed in which, by default, the first tests are first administered and wherein only responsive to a score for the first tests being outside of a normal range are the set of second tests then administered. This hence applies an initial filter to identify subjects who are more likely potential sufferers of subtle cognitive impairment. For example, in some embodiments, a procedure can be followed in which, by default, timed tests are first administered and wherein only responsive to a score for the timed tests being outside of a normal range are a set of untimed tests then administered.

Thus, in some embodiments, at least a subset of the battery of cognitive tests are configurable to either impose or not impose a time-constraint or other cognitive demand constraint when administering the test; and wherein the method comprises: first administering a cognitive test with a time constraint or other cognitive demand constraint, to form one of the one or more first tests; determining a score for said test; and determining whether said score is within a pre-defined normal range. In some embodiments, responsive to the score being outside of the pre-defined normal range (e.g. below a threshold), the method further comprises administering the same cognitive test without the time-constraint or other cognitive demand constraint imposed, to form one of the one or more second tests.

In some embodiments, tests can be performed with additional imposed layers of cognitive demand, e.g. presence of environmental noise, and imposition of a multitasking requirement.

Thus in some embodiments, at least a subset of the battery of cognitive tests are configurable to impose one or more additional cognitive demand parameters/constraints.

In some embodiments, the method additionally comprises: administering at least a subset of the battery of cognitive tests a further time with an additional one or more cognitive demand parameters/constraints added.

In some embodiments, the method comprises administering at least a subset of the battery of cognitive tests at least a further two more times, each time with a different number of the additional cognitive demand parameters/constraints added;

In some embodiments, the method comprises computing said characteristic value separately for scores of each administration of the battery of tests.

In some embodiments, the method comprises determining a relationship between the characteristic value and a number of cognitive demand parameters/constraints added. In some embodiments, the detecting the presence of subtle cognitive impairment is based at least in part on said relationship.

In some embodiments, the one or more additional cognitive demand parameters/constraints include one or more of: presence of environmental noise, imposition of a multitasking requirement, adjustment of visibility of test stimuli (e.g. brightness, clearness of stimuli, contrast of stimuli), imposition of tactile constraints (e.g. chair moving, pen difficult to hold).

In some embodiments, the scores of tests might be calibrated or normalized according to a level of background noise in the environment. This ensures that the scores are comparable to one another in terms of the cognitive demand level imposed in each by background noise.

Thus, in some embodiments, the method further comprises: sensing an environmental noise intensity during the administering of the battery of cognitive tests. In some embodiments, the method comprises calibrating or compensating the derived scores for the battery of tests to control for the sensed environmental noise. Additionally or alternatively, in some embodiments, the method comprises generating an advice item for communication to an operator indicative of the detected presence and intensity of environmental noise.

For example environmental noise level or intensity could be detected using cameras (and application of a machine vision algorithm to estimate noise) and/or microphones.

In some embodiments, the system further comprises a memory storing a database which comprises control instructions for administering each of the battery of cognitive tests.

In some embodiments, the system further comprises a test apparatus for administering a digital neuropsychological assessment to a subject.

The invention can also be embodied in the form of a method.

Thus, embodiments of the invention also include a method which comprises:
controlling a test apparatus to administer a battery of cognitive tests to a subject, wherein the battery of cognitive tests includes:
   a plurality of first tests, wherein each of the first tests is a test for which a time constraint is imposed related to a completion time for the test or for which another cognitive demand constraint is imposed, and
   a plurality of second tests, wherein each of the second tests is a test for which said time constraint is not imposed or said other cognitive demand constraint is not imposed;
generating a first composite score from a combination of test scores obtained for the subject from the plurality of first tests;
generating a second composite score from a combination of test scores obtained for the subject from the plurality of second tests;
computing a characteristic value based on the first composite score and the second composite score;
detecting presence of subtle cognitive impairment based on the characteristic value; and
generating an output indicative of the detection.

The invention can also be embodied in the form of software. Thus, embodiments of the invention also include a computer program product comprising code means, configured, when run on a processor, to cause the processor to perform a method in accordance with any embodiment or example described in this disclosure. It is also noted that there is intended to be complete compatibility in features between the different aspects of the invention (system, method, computer program product), so that features or options recited in the context of one aspect can be applied equally to any other aspect.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 schematically outlines components of an example processing arrangement and system in accordance with one or more embodiments of the invention;
Fig. 3 shows a graph illustrating comparative performance scores for healthy subjects versus those with subtle cognitive impairment for timed and untimed tests; and
Fig. 4 shows a graph illustrating a ratio between composite scores of untimed to timed cognitive tests as a function of the number of cognitive demands imposed, for healthy subjects and subjects with subtle cognitive impairment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a means for distinguishing subjects with subtle cognitive impairment in the context of a neuropsychological assessment which comprises a battery of cognitive tests. A first set of tests is administered which have a time-constraint or other cognitive demand constraint/parameter imposed. A second set of tests is administered for which the time constraint or other cognitive demand constraint/parameter is not imposed. Based on comparing or relating the scores between the two sets of tests, subtle cognitive impairment can be detected, based on a criterion that subjects with subtle cognitive impairment present with a greater disparity in these two sets of scores than do normal subjects.

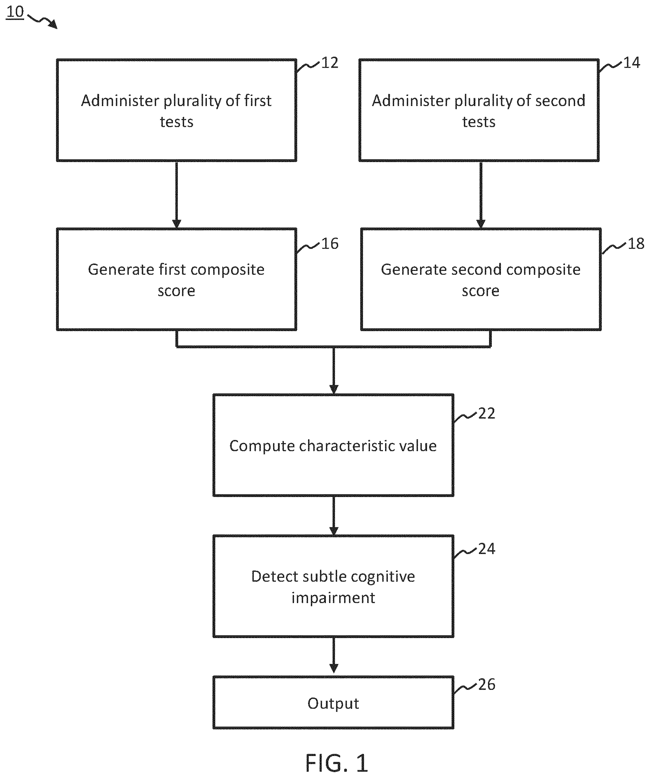

Fig. 1 outlines in block diagram form steps of an example method according to one or more embodiments. The steps will be recited in summary, before being explained further in the form of example embodiments.

The method 10 comprises controlling a test apparatus to administer a battery of cognitive tests to a subject. The battery of cognitive tests which is administered includes: a plurality of first tests 12, wherein each of the first tests is a test for which a time constraint is imposed related to a completion time for the test or for which another cognitive demand constraint is imposed, and a plurality of second tests 14, wherein each of the second tests is a test for which said time constraint is not imposed or said other cognitive demand constraint is not imposed.

The method further comprises generating 16 a first composite score from a combination of test scores obtained for the subject from the plurality of first tests. The method further comprises generating 18 a second composite score from a combination of test scores obtained for the subject from the plurality of second tests. The method further comprises computing 22 a characteristic value based on the first composite score and the second composite score (e.g. the difference between the scores or a ratio between the scores). The method further comprises detecting 24 presence of subtle cognitive impairment based on the characteristic value. The method further comprises generating 26 an output indicative of the detection. For example the output may be a data output for communication to a further device such as a memory, a further computing device, or a user interface device.

As noted above, the method can also be embodied in software. Thus, another aspect of the invention is a computer program product comprising code means, configured, when run on a processor, to cause the processor to perform a method in accordance with any embodiments outlined in this document, or in accordance with any claim of this application.

As noted above, the method can also be embodied in hardware form, for example in the form of a system which is configured to carry out a method in accordance with any example or embodiment described in this document, or in accordance with any claim of this application.

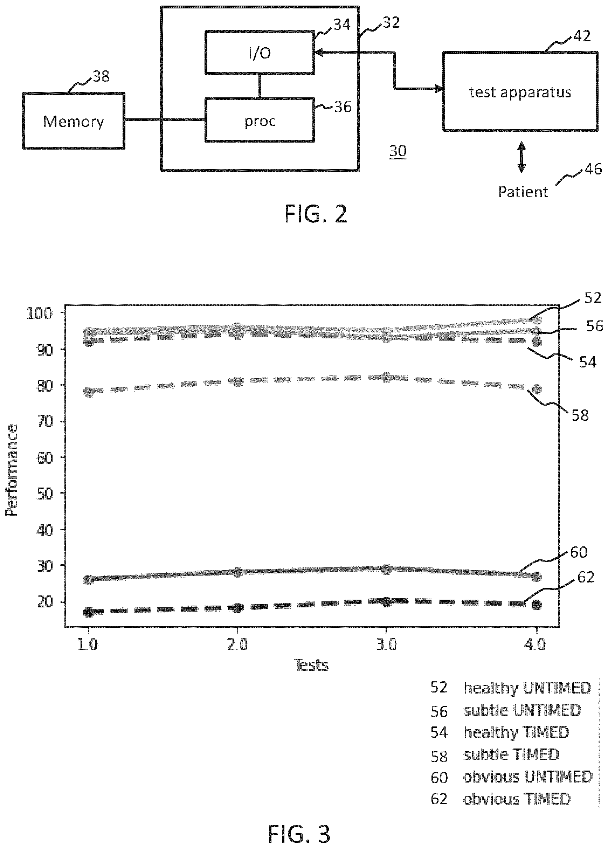

To further aid understanding, Fig. 2 presents a schematic representation of an example system 30 configured to execute a method in accordance with one or more embodiments of the invention.

The illustrated system comprises a processing unit 32 comprising an input/output 34 and one or more processors 36.

The one or more processors are adapted to perform a method in accordance with the steps already recited above, or in accordance with any embodiment described in this document.

More concretely, in some embodiments, the one or more processors 36 are adapted to execute a method which comprises: controlling, via control signals communicated though the input/output 34, a test apparatus 42 to administer a battery of cognitive tests to a subject. The battery of cognitive tests includes: a plurality of first tests, wherein each of the first tests is a test for which a time constraint is imposed related to a completion time for the test or for which another cognitive demand constraint is imposed, and a plurality of second tests, wherein each of the second tests is a test for which said time constraint is not imposed or said other cognitive demand constraint is not imposed. The method executed by the one or more processors 36 further includes generating a first composite score from a combination of test scores obtained for the subject from the plurality of first tests; generating a second composite score from a combination of test scores obtained for the subject from the plurality of second tests; computing a characteristic value based on the first composite score and the second composite score; detecting presence of subtle cognitive impairment based on the characteristic value; and generating an output at the input/output 34 indicative of the detection.

An aspect of the invention is a system comprising just the processing unit 32 (or expressed another way, just the input/output 34 and the one or more processors 36).

Optionally, the system 30 can further comprise a memory 38 storing a database which comprises control instructions for administering each test of the battery of cognitive tests.

Optionally, the system may comprise a test apparatus 42 for administering a battery of cognitive tests to a subject. For example, this may be a computer-based testing apparatus, i.e. comprising a computing device. It may be a dedicated computing console or terminal. It may comprise a mobile computing device such as a tablet computer, smartphone or laptop. It may include a user interface. The user interface may include one or more sensory output devices for generating user-perceptible stimuli. The user interface may include a user input apparatus. This may include one or more pointer devices, such as an electronic pen or pencil, and/or a touchscreen, and/or a gesture capture device such as a handheld unit with an inertial measurement unit integrated therein or a camera/sensor based gesture capture device.

The principles behind embodiments of the invention will now be explained further.

It is expected that healthy people, people with subtle cognitive impairment, e.g. caused by mild traumatic brain injury (mTBI) or multiple sclerosis (MS), and people with obvious cognitive impairment (e.g. dementia, mild or severe TBI) will exhibit different profiles in respect of the two (i.e. timed and untimed) composite scores.

This is depicted in Fig. 3 which is illustrative of expected results. Each line represents a composite performance score (y-axis) composed from different numbers of tests (x-axis). Each composite score could be formed for example from an average of a set of scores.

Line 52 corresponds to a healthy subject for untimed tests (without a time constraint imposed). Line 54 corresponds to a healthy subject for timed tests (with a time constraint imposed). Line 56 corresponds to a subject with subtle cognitive impairment for untimed tests. Line 58 corresponds to a subject with subtle cognitive impairment for timed tests. Line 60 corresponds to a subject with obvious (i.e. non-subtle) cognitive impairment for untimed tests. Line 62 corresponds to a subject with obvious cognitive impairment for timed tests. For all classes of subject, performance is slightly worse on timed cognitive tests than on untimed cognitive tests. While for both healthy and subtly impaired people, performance on untimed tests might be high and comparable, the disparity between timed and untimed tests should be larger for people with subtle cognitive impairment than for healthy people and people with obvious cognitive impairment.

Purely by way of example, the Philips ISC (IntelliSpace Cognition) platform includes the following cognitive tests which do not impose a time-constraint. Thus, the plurality of second tests may include any two or more of these in some examples:
Clock Drawing Test
Digit Span Test
Rey Auditory Verbal Learning Test
Rey Osterrieth Complex Figure Test

For further details of these tests, reference is made to the book: Lezak, M. D., Howieson, D. B., Bigler, E. D., & Tranel, D. (2012). Neuropsychological assessment (5th ed.). New York, US: Oxford University Press.

By way of further development of the example, the Philips ISC (IntelliSpace Cognition) platform includes the following cognitive tests which do impose a time-constraint. Thus, the plurality of first tests may include any two or more of these in some examples:
Trail Making Test
Controlled Oral Word Association Test
Category Fluency Test
(Star Cancellation Test)
Symbol Digit Modalities

For further details of these tests, reference is made to the book: Lezak, M. D., Howieson, D. B., Bigler, E. D., & Tranel, D. (2012). Neuropsychological assessment (5th ed.). New York, US: Oxford University Press.

As already indicated, a first composite score is generated from a combination of test scores obtained for the subject from the plurality of first tests and a second composite score is generated from a combination of test scores obtained for the subject from the plurality of second tests. These composite scores may for example each be formed as an average of scores for the set of untimed tests and the set of timed tests respectively. A weighted average could also be used in some examples.

The method further comprises computing a characteristic value based on the first composite score and the second composite score. This may be a value which is indicative of a relationship between the first and second scores. It may be a value which is correlated with a degree of disparity between the two scores.

A simple example of a characteristic value is a ratio between the first composite score and the second composite score, or vice versa.

Thus, in some embodiments, based on automatically computed first and second composite scores, ratio or difference scores can be calculated for every patient. The ratio or difference score provides the characteristic value.

By way of example, in some embodiments, based on automatically computed TIMED and UNTIMED composite scores, ratio or difference scores can be calculated for every patient. The ratio or difference score provides the characteristic value.

For example, a ratio of first/second (e.g. TIMED/UNTIMED) composite scores can be expected to be lower for patients with subtle cognitive impairment than for patients who are normal or healthy (where "/" here means 'divided by"). The ratio of second/first (UNTIMED/TIMED) composite scores can be expected to higher for patients without subtle cognitive impairment.

If the ratio score (e.g. TIMED/UNTIMED) falls below a certain threshold for a patient who scored within the norms on the individual cognitive tests, it is more likely that the patient has subtle cognitive impairment. By way of illustration, Table 1 below shows an illustration of composite scores for a healthy person and a person with subtle cognitive impairment, and also shows, by way of illustration, a TIMED:UNTIMED composite score ratio. Note, this is not based on real data.

**Table 1**

| | **Healthy individual** | **Person with subtle cognitive impairment** |
|---|---|---|
| **Composite score TIMED** | 92 | 86 |
| **Composite score UNTIMED** | 98 | 95 |
| **Ratio (TIMED:UNTIMED)** | 0.94 | 0.91 |

By computing TIMED and UNTIMED composite scores and TIMED/UNTIMED ratio or difference scores, it is possible to better detect subtle cognitive impairment, even if patients with subtle cognitive impairment perform within the norm on individual cognitive tests.

For example, if for a healthy person the composite score for the untimed tests was 95 and for the timed tests it was 90, then this would yield a TIMED:UNTIMED ratio score of 90/95 = 0.95. If for a person with subtle cognitive impairment, the score for the untimed tests was 93 and for the timed tests it was 82, this would result in a TIMED:UNTIMED ratio score of 82/93 = 0.88, which is smaller than the TIMED:UNTIMED ratio score for the healthy person because there is a larger difference between the two composite scores. Thus, in some embodiments, subtle cognitive impairment may be detected in the event that a ratio between the first (TIMED) composite score and the second (UNTIMED) composite score falls below a pre-defined threshold. Likewise, the same principles apply in reverse, so that subtle cognitive impairment may be detected in the event that a ratio between the second (UNTIMED) composite score and the first (TIMED) composite score is above a pre-defined threshold.

Optionally, each of the test scores which form the first composite score, and each of the tests scores which form the second composite scores may be normalized or weighted so that the results of one test are comparable to another. This can be done based on historical test data for healthy populations and populations with pathology, from which test data a 'norm' score or norm range can be established which represents a threshold or range outside of which scores are likely to indicate abnormality. Then, the scoring system for each test can be calibrated so that for example all the tests run across a standardized score range (e.g. 1-100), and wherein there is standardization of the norm score across this range for every test. This way, a score for one test is comparable to a score from another test in terms of its relationship to the 'norm'.

However, this is optional in the context of the present invention because in fact the characteristic value is based on a relationship between two composite scores, and thus it is based on relative values of two sets of tests (e.g. timed and untimed). Therefore absolute score values may not be essential.

In some embodiments, it is proposed that cognitive tests be conducted with and without time pressure to increase or decrease cognitive demands, respectively. Even tests that are normally untimed (e.g. the Rey Auditory Verbal Learning Test) could be conducted in a timed way and, likewise, tests that are normally timed (e.g. the Trail Making Test) could be conducted in an untimed way. Adding time pressure to an untimed cognitive test increases the cognitive demands of the test and thus might make it more likely to detect subtle cognitive impairment. Removing time pressure from a timed test decreases the cognitive demands of the test and might make it easier for the patient to perform the test. In addition, this approach has the effect of providing a purer measure of the impact of the timing upon performance, because the characteristics of the two tests would all be the same except for the presence or absence of the time constraint.

It is noted that, for each version of the test - with or without the time constraint - a separate norm score might be established in advance, to allow for standardization with other test scores in the battery.

The same principle can be applied for any other cognitive demand constraint aside from a time constraint: the same test can be applied with or without the constraint to form one of the first and second tests respectively.

Thus, following this concept, in some embodiments, at least a subset of the battery of cognitive tests may be configurable to either impose or not impose a time-constraint or other cognitive demand constraint when administering the test; and wherein the method comprises administering a same cognitive test with a time-constraint or other cognitive demand constraint imposed, to form one of the one or more first tests, and without the time constraint or other cognitive demand constraint imposed, to form one of the one or more second cognitive tests.

Additionally or alternatively, in some embodiments, performance on the first tests can be used to personalize the battery of tests administered as part of the neuropsychological assessment, and guide the selection of subsequent cognitive tests to administer. For instance, if a patient with suspected subtle cognitive impairment performs well on a first test with the cognitive demand constraint present (e.g. a timed cognitive test), i.e. within the norm, subsequent tests could be applied with the same cognitive demand constraint imposed, e.g. timed, (even if the normal version of the tests were without any time constraint) in order to increase cognitive demands. If in contrast a patient with suspected subtle cognitive impairment performs poorly on a first test with the cognitive demand constraint present cognitive test (i.e. outside the norm), subsequent tests could be applied without the cognitive demand constraint imposed, e.g. untimed (even if their original version is timed) in order to decrease cognitive demands.

To state this in other words, it is proposed in some embodiments that at least a subset of the battery of cognitive tests are configurable to either impose or not impose a time-constraint or other cognitive demand constraint when administering the test, and wherein the method comprises the following:
First administering a cognitive test with a time constraint or other cognitive demand constraint, to form one of the one or more first tests;
determining a score for said test;
determining whether said score is within a pre-defined normal range (e.g. above a minimum threshold), and
responsive to the score being outside of the pre-defined normal range, administering the same cognitive test without the time-constraint or other cognitive demand constraint imposed, to form one of the one or more second tests.

Additionally or alternatively to any of the above, in some embodiments, one or more additional cognitive demands may be added to one or multiple cognitive tests to increase their difficulty (i.e. two or more cognitive demands might be applied instead of just one). As already discussed, time pressure is one way to make a test more cognitively demanding. Other factors that increase the cognitive demands of a test besides time pressure include, among others, environmental noise or multitasking. In this regard, reference is made for example to the paper: Reynolds, McClelland & Furnham (2014). An investigation of cognitive test performance across conditions of silence, background noise and music as a function of neuroticism. Anxiety, Stress & Coping, 27, 410-421.

Adding several cognitive demands has a cumulative effect on test performance. Patients with subtle cognitive impairment are less likely to cope with this increased difficulty than healthy people. Thus, performance on a cognitive test may be more informative when considering how many different cognitive demands a test contains, e.g. by calculating a ratio and the trend in ratios and potentially comparing these to a healthy population.

From this consideration, it is proposed that in some embodiments, at least a subset of the battery of cognitive tests are configurable to impose one or more additional cognitive demand parameters and wherein the method additionally comprises: administering at least a subset of the battery of cognitive tests a further time with an additional one or more of the cognitive demand parameters added.

To achieve comparative indicators between patients which take account of differential performance with different numbers of cumulative cognitive demands added (in accordance with the concept proposed above), in some embodiments, the following may be implemented. The method may comprise:
administering at least a subset of the battery of cognitive tests at least a further two more times, each time with a different number of the additional cognitive demand parameters added;
computing said characteristic value separately for scores of each administration of the battery of tests;
determining a relationship between the characteristic value and a number of cognitive demand parameters added; and
optionally detecting the presence of subtle cognitive impairment based at least in part on said relationship.

As mentioned, and by way of example, the one or more additional cognitive demand parameters may include one or more of: presence of environmental noise, and imposition of a multitasking requirement.

To illustrate this concept, Table 2 below presents an illustrative set of comparative data (not based on real data).

**Table 2**

| **Number of cognitive demands in test** | **People with subtle cognitive impairment** | | **Healthy people** | |
|---|---|---|---|---|
| | **Performance normalized** | **Difference (ratio)** | **Performance normalized** | **Difference (ratio)** |
| 1 | 12.2 | - | 12.2 | - |
| 2 | 11.0 | 1.2 (1.109) | 11.9 | 0.3 (1.025) |
| 3 | 9.6 | 1.4 (1.146) | 11.3 | 0.6 (1.053) |
| 4 | 7.3 | 2.3 (1.315) | 10.8 | 0.5 (1.046) |
| ... | 3.5 | 3.8 (2.086) | 10.1 | 0.7 (1.069) |

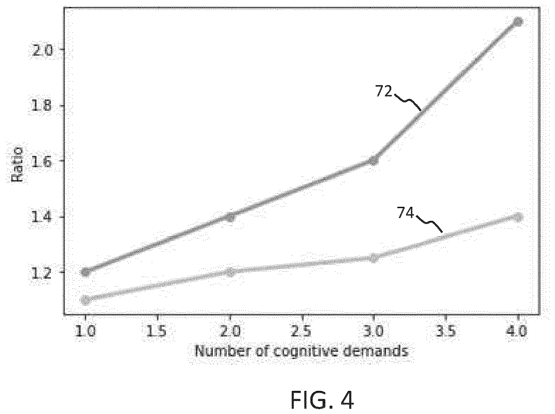

This illustrates the expectation that for both healthy people and people with subtle cognitive impairment, the normalized performance decreases with additional cognitive demands. However, it can be seen that normalized performance decreased much more markedly for individuals with subtle cognitive performance. The consequence is that a disparity (difference) between scores for the second and first sets of tests increases more steeply with the number of cognitive demands for people with subtle cognitive impairment than for people who are healthy. Likewise a ratio between composite scores for second:first tests (e.g. untimed:timed tests) also increases more steeply with number of cognitive demands added for people with subtle cognitive impairment than for a healthy population. This is illustrated in Fig. 4, in which line 72 corresponds to subjects with subtle cognitive impairment and line 74 corresponds to healthy subjects. The x-axis corresponds to the number of cognitive demands, and the y-axis corresponds to a ratio of the composite scores for untimed:timed tests.

Thus if this ratio for example is used as the characteristic value, it can be seen that a relationship between the characteristic value (y-axis) and the number of cognitive demand parameters added (x-axis) is different for subjects with subtle cognitive impairment compared to healthy subjects.

Of course, the imposing of additional cognitive demand parameters could be done as an optional feature by itself, without considering the relationship shown in Fig. 4. As is apparent from Fig. 4, the imposition of additional cognitive demand parameters leads to a greater disparity between the second:first score ratio for subtle cognitive impairment subjects and healthy subjects. Thus, sensitivity of detection of subtle cognitive impairment is improved by imposing additional cognitive demands.

In some embodiments, the presence and intensity of environmental noise during the performance of a cognitive test (battery) is determined in order to be able to better control for environmental noise. This is important particularly in cases where environmental noise is added as an additional cognitive demand.

Thus, by way of example, this may comprise: sensing an environmental noise intensity during the administering of the battery of cognitive tests, and then calibrating or compensating the derived scores for the battery of tests to control for the sensed environmental noise and/or generating an advice item for communication to an operator indicative of the detected presence and intensity of environmental noise.

Thus, in this set of embodiments, a baseline environmental noise level is determined. This allows for determining a comparative level of test difficulty. It also allows for adding additional environmental noise as an additional cognitive demand in a standardized way, i.e. so that the added environmental noise imposes a standard additional level of difficulty.

A level of environmental noise could be measured using one or more microphones in some examples, or even using cameras, the image data from which could be processed using machine vision algorithms adapted to infer noise levels from image data.

More generally, an environmental observation system comprising one or more cameras and/or microphones may be used to obtain an indication of context. This information could subsequently be used for advising or coaching test administrators in adapting or changing the context.

In some embodiments, a user interface may be controlled to display an advice item advising to leave out data points recorded under compromised environmental circumstances, e.g. high background noise levels, improper seating of subject, improper position and/or motion of an input device (e.g. tablet computer) on which the cognitive test is conducted; or presence of other distractors.

In some embodiments, a real-time reliability score may be computed for each cognitive test result based on acquired environmental context data. Based on this score, one or more advice items may be generated and presented on a user interface advising one or more response actions: e.g. stop test or redo test. In some embodiments, dependent on the reliability score falling below a pre-defined threshold, the score may be discounted from the computation of the composite score.

In some embodiments, in relation to the context recording system, event-based recording may be implemented. Here, to ensure a subject's privacy, context data might only be recorded within a set time window, e.g. starting a set time interval before a cognitive test battery, finishing a set time interval afterward. Context data might be automatically deleted after it has been used for computing the reliability scores.

Embodiments of the invention described above employ one or more processors. The one or more processors may be located in a single containing device, structure or unit, or may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The one or more processors can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system comprising:
an input/output; and
one or more processors adapted to execute a method, the method comprising:
controlling, via control signals communicated though the input/output, a test apparatus to administer a battery of cognitive tests to a subject, wherein the battery of cognitive tests includes:
a plurality of first tests, wherein each of the first tests is a test for which a time constraint is imposed related to a completion time for the test or for which another cognitive demand constraint is imposed, and
a plurality of second tests, wherein each of the second tests is a test for which said time constraint is not imposed or said other cognitive demand constraint is not imposed;
generating a first composite score from a combination of test scores obtained for the subject from the plurality of first tests;
generating a second composite score from a combination of test scores obtained for the subject from the plurality of second tests;
computing a characteristic value based on the first composite score and the second composite score;
detecting presence of subtle cognitive impairment based on the characteristic value; and
generating an output at the input/output indicative of the detection.

2. The system of claim 1, wherein each of the first tests is a test for which a time constraint is imposed related to a completion time for the test, and wherein the time-constraint comprises an instruction to the subject to complete the task as fast as possible, and/or comprises a time limit for completion of the task.

3. The system of claim 1 or 2, wherein the characteristic value comprises a ratio between the first composite score and the second composite score, or vice versa.

4. The system of claim 3, wherein subtle cognitive impairment is detected in the event that the ratio between the first composite score and the second composite score falls outside of a pre-defined range, for example falls below a pre-defined threshold.

5. The system of any of claims 1-4, wherein the one or more second tests include one or more of: Clock Drawing Test, Digit Span Test, Rey Auditory Verbal Learning Test, Rey Osterrieth Complex Figure Test.

6. The system of any of claims 1-5, wherein each of the first tests is a test for which a time constraint is imposed related to a completion time for the test, and wherein the one or more first tests include one or more of: Trail Making Test, Controlled Oral Word Association Test, Category Fluency Test, Star Cancellation Test, Symbol Digit Modalities.

7. The system of any of claims 1-6, wherein at least a subset of the battery of cognitive tests are configurable to either impose or not impose a time-constraint or other cognitive demand constraint when administering the test; and
wherein the method comprises administering a same cognitive test with a time-constraint imposed, to form one of the one or more first tests, and without the time constraint or other cognitive demand constraint imposed, to form one of the one or more second cognitive tests.

8. The system of any of claims 1-7,
wherein at least a subset of the battery of cognitive tests are configurable to either impose or not impose a time-constraint when administering the test; and
wherein the method comprises:
first administering a cognitive test with a time constraint or other cognitive demand constraint, to form one of the one or more first tests,
determining a score for said test,
determining whether said score is within a pre-defined normal range, and
responsive to the score being outside of the pre-defined normal range, administering the same cognitive test without the time-constraint or other cognitive demand constraint imposed, to form one of the second tests.

9. The system of any of claims 1-8,
wherein at least a subset of the battery of cognitive tests are configurable to impose one or more additional cognitive demand constraints; and
wherein the method additionally comprises:
administering at least a subset of the battery of cognitive tests a further time with an additional one or more of the cognitive demand constraints added.

10. The system of claim 9, wherein:
the method comprises administering at least a subset of the battery of cognitive tests at least a further two more times, each time with a different number of the additional cognitive demand constraints added;
computing said characteristic value separately for scores of each administration of the battery of tests;
determining a relationship between the characteristic value and a number of cognitive demand constraints added; and
wherein the detecting the presence of subtle cognitive impairment is based at least in part on said relationship.

11. The system of claim 9 or 10, wherein the one or more additional cognitive demand constraints include one or more of: presence of environmental noise, and imposition of a multitasking requirement.

12. The system of any of claims 1-11, wherein the method further comprises:
sensing an environmental noise intensity during the administering of the battery of cognitive tests; and
calibrating or compensating the derived scores for the battery of tests to control for the sensed environmental noise; and/or
generating an advice item for communication to an operator indicative of the detected presence and intensity of environmental noise.

13. The system of any of claims 1-12, further comprising a test apparatus for administering the batter of cognitive tests to the subject.

14. A computer implemented method comprising:
controlling a test apparatus to administer a battery of cognitive tests to a subject, wherein the battery of cognitive tests includes:
a plurality of first tests, wherein each of the first tests is a test for which a time constraint is imposed related to a completion time for the test or for which another cognitive demand constraint is imposed, and
a plurality of second tests, wherein each of the second tests is a test for which said time constraint is not imposed or said other cognitive demand constraint is not imposed;
generating a first composite score from a combination of test scores obtained for the subject from the plurality of first tests;
generating a second composite score from a combination of test scores obtained for the subject from the plurality of second tests;
computing a characteristic value based on the first composite score and the second composite score;
detecting presence of subtle cognitive impairment based on the characteristic value; and
generating an output indicative of the detection.

15. A computer program product comprising code means, configured, when run on a processor, to cause the processor to perform the method of claim 14.
